(19) Europäisches Patentamt
European Patent Office
Office européen des brevets

(11) **EP 4 742 252 A1**

(12) **EUROPEAN PATENT APPLICATION**

(43) Date of publication:
**13.05.2026   Bulletin 2026/20**

(21) Application number: **24212124.2**

(22) Date of filing: **11.11.2024**

(51) International Patent Classification (IPC):
**G16C 60/00** (2019.01)   **G16B 5/30** (2019.01)
**G01N 33/49** (2006.01)

(52) Cooperative Patent Classification (CPC):
**G16B 5/30; G01N 33/4925; G16C 20/10;**
**G16C 20/20**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Aalborg Universitet**
**9220 Aalborg Øst (DK)**

(72) Inventors:
• **Rees, Stephen Edward**
 **9260 Gistrup (DK)**
• **Thomsen, Lars Pilegaard**
 **9520 Skørping (DK)**
• **Nevirian, Bahareh**
 **9220 Aalborg Øst (DK)**
• **Fagerberg, Steen Kåre**
 **9400 Nørresundby (DK)**

(74) Representative: **Plougmann Vingtoft a/s**
**Strandvejen 70**
**2900 Hellerup (DK)**

(54) **A METHOD FOR COMPENSATING THE EFFECTS OF TIME DELAY FROM SAMPLING TO MEASUREMENT IN A BLOOD SAMPLE**

(57)    The invention relates to a method for compensating the effects of time delay (TD) between sampling and actual measurement of acid-base, oxygenation and/or electrolyte status in a blood sample from a subject, such as a human patient. A mathematical model is simulating time development in the blood sample, the mathematical model having 1) a first sub-model describing red blood cell metabolism, and 2) a second sub-model describing the effect of the red blood cell metabolism on the whole blood acid-base chemistry. The mathematical model calculates from a time of measurement (Tm) to the time of sampling (Ts) a modified set of blood variables to compensate for a time delay (TD), e.g. by calculating backwards in time 0.5-3 hours. The invention can simulate the biochemical processes occurring in blood, and demonstrates the ability to simulate changes in measured blood variables over time. This may simplify transport and/or storage constraints of blood samples, and improve quality and accuracy of blood gas measurements.

FIG. 3

**EP 4 742 252 A1**

**Description**

FIELD OF THE INVENTION

The present invention relates to a computer-implemented method for compensating the effects of time delay (TD) between sampling and actual measurement of acid-base, oxygenation and/or electrolyte status in a blood sample. The invention also relates to a corresponding system for measurement of blood, a corresponding computer programme product, and corresponding use of the method and/or the system.

BACKGROUND OF THE INVENTION

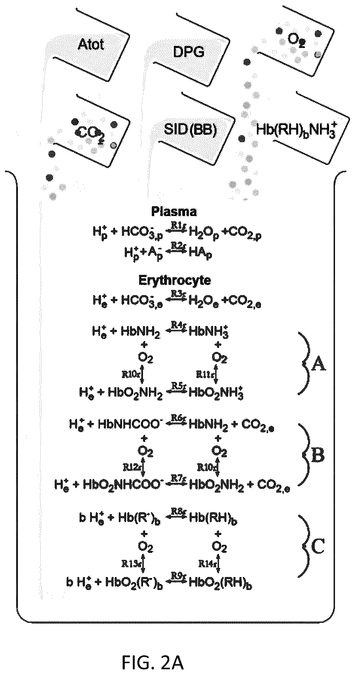

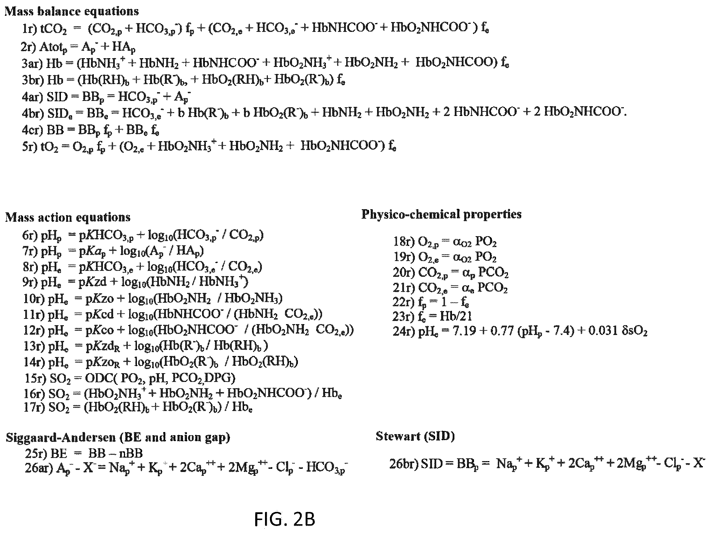

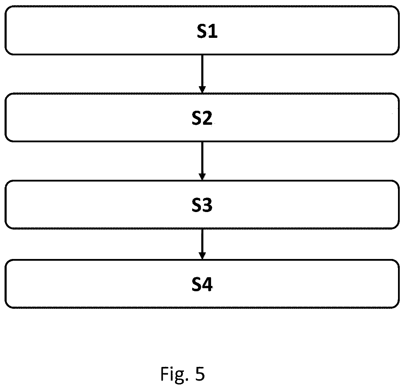

Measurements of blood acid-base status are usually performed quickly after blood sampling to avoid errors and to minimize physiological changes before sampling. This necessitate rapid transport of samples and therefore may be problematic in situations where the patient is far from the analyser, either because the patient is far from a clinic having appropriate blood measurement facilities - or because transportation and/or working procedures in a large hospital makes it difficult to transport the sample from the patient to the point of blood measurement in the hospital quickly enough.
Thus, it is generally accepted in this field that measurements of acid-base, oxygenation, and electrolyte status of blood should be performed quickly after sampling, and typically with a maximum delay between 25 to 40 minutes. This may present clinical problems if blood gas analysis devices are remote from sampling site such as in pre-hospital or telemedical settings, ambulance transport or where delays in processing are inherent, such as taking of samples from multiple patients on a ward round. Most studies emphasize the unsuitability of results obtained from blood samples analyzed after the allowable storage time, and postulate mechanisms of change of individual variables.
US 6,859,738 to Becton, Dickinson and Company suggests a solution to the delay problem with e.g. blood measurements. Thus, a method is provided for estimating the initial level of an analyte in a blood sample based on an actual level observed, a time of storage of the blood sample, a storage temperature, and a type of container in which said blood sample is stored. Equations are presented for a particular analyte based on observations of actual analyte levels initially after a blood sample is drawn, as well as at various times thereafter. The time of storage, the temperature of storage, and the type of container are observed along with actual analyte level in each observation. These equations are considered useful if the estimated initial values are more accurate than the actual observed final values. The equations thereby represent the time development of blood parameters, which is made based on collected data, cf. Figs. 2 & 5 for some examples. However, these equations are based on purely statistical tests and models, such as a polynomial regression model with the square of the time and the square of the storage temperature. For systems where variables are tightly related by physiochemical processes, modelling using statistical approaches may not be appropriate or even adequate. Blood oxygen, acid-base and electrolytes are such a system, where values are tightly coupled and changes over time require consideration of the whole system for quantifying the effects of delay in blood analysis.
Hence, an improved method for compensating the effects of time delay (TD) from sampling to actual measurement of for example acid-base, oxygenation and/or electrolyte status in a blood sample would be advantageous, and in particular a more efficient and/or reliable method for compensating this time delay would be advantageous.

OBJECT OF THE INVENTION

It is a further object of the present invention to provide an alternative to the prior art.
In particular, it may be seen as an object of the present invention to provide a method that solves the above-mentioned problems of the prior art having for example an approach based on statistical analysis of blood samples measured over time.

SUMMARY OF THE INVENTION

Thus, the above described object and several other objects are intended to be obtained in a first aspect of the invention by providing a computer-implemented method for compensating the effects of time delay (TD) between sampling and actual measurement of acid-base, oxygenation and/or electrolyte status in a blood sample from a subject, the method comprising:

- providing a blood sample with a known or estimated time of sampling (Ts),

- performing a blood gas measurement of said blood sample at a later time of measurement (Tm) resulting in a set of blood variables (BV'[Tm]) indicative of acid-base, oxygenation and/or electrolyte status in said blood sample,

- providing a mathematical model based on a plurality of physiological sub-models simulating time development in blood, the mathematical model comprising:

  o a first sub-model describing red blood cell metabolism, and
  o a second sub-model describing the effect of said red blood cell metabolism on the whole blood acid-base chemistry, and

- applying said mathematical model to calculate from the time of measurement (Tm) to the time of sampling (Ts) a modified set of blood variables (BV[Ts]) indicative of the corresponding acid-base, oxygenation and/or electrolyte status of said blood sample so as to at least partly compensate for said time delay (TD) between sampling and actual measurement.

The invention is particularly, but not exclusively, advantageous for obtaining a method to simulate the biochemical processes occurring in blood, and demonstrate the ability of this mathematical model to simulate changes in measured blood variables over time. The core of the mathematical model is based on the physiological effects caused by anaerobic metabolism of red blood cells (RBCs) comprising for example glucose utilization, lactate, and strong acid production; but also comprises the acid-base chemistry of blood, and the distribution of electrolytes between plasma and erythrocyte factions according to biochemical equations presented below.

Definitions:

In the context of the present invention, it's to be understood that the time of sampling (Ts) and the time of measurement (Tm) can be known, or equivalently or alternatively the time difference between the time of sampling (Ts) and the time of measurement (Tm) i.e. the time delay (TD) can be known, estimated or calculated, and used in the computer-implemented method for compensating the effects of time delay (TD) between sampling and actual measurement of acid-base, oxygenation and/or electrolyte status in a blood sample according to the present invention. Thus, the skilled person in blood gas measurements will readily understand that a method for compensating or mitigating the time delay may be used for both possibilities. If the time delay is uncertain, the present invention may also be applied for modelling one, or more, blood variable(s) over a time interval corresponding to said uncertainty, thereby yield a corresponding interval of the blood variable(s). Further, when referring to a set of blood variables, it may also as a special case be just one blood variable, which is measured and where the time delay is compensated by the present invention.

In the context of the present invention, it's to be understood that the provided blood sample may be prepared by a professional health person working in a clinic or a hospital, such as a nurse or a doctor, and that the actual taking of the blood sample does not form part of the claimed invention, but the blood sample is nevertheless used as an input sample for further analysis in a dedicated blood gas measurement apparatus or system. The present invention may be applied for various blood samples in, or near, a clinical environment or a hospital, including but not limited to these types of blood samples:

- Arterial Blood: Collected from oxygen-rich blood that is pumped from the heart through the arteries to the rest of the body.

- Capillary Blood: This sample is obtained from an ear prick, finger prick or heel prick (in infants) and is often used for quick tests, such as blood glucose monitoring.

- Venous Blood: Collected from a vein, this is the most common type of blood sample used for a wide range of tests, including metabolic panels and blood cultures.

For each of these blood samples, it is common to evaluate blood gas, acid-base and electrolyte status etc., and the present invention may be applied for compensating a time delay between sampling and measurement of these blood variables.

Further, it is particularly contemplated that present invention can be combined with another mathematical model for compensating a contamination of a blood sample between sampling and blood gas measurements, particular accounting for gas or air contamination of a blood sample taken in vacuum tube where a residual gas volume will be present.

In the context of the present invention, it's to be understood that a mathematical model may be an abstract representation of a real-world system using mathematical concepts and language. It may serve to describe, analyze, and predict the behaviour of that system. The process of creating such a model is known as mathematical modelling as the skilled person in modelling will readily understand. In the context of the present invention, it's further to be understood that a physiological model may be a mathematical or computational representation that simulates the biological, chemical or other physical functions and processes of living organisms or systems. Such physiological models or sub-models may be used to understand, predict, and analyze how various physiological systems operate under different conditions. In particular the present invention applies a mathematical model comprising a plurality of physiological sub-models for describing the effects of time delay between sample and measurement of a blood sample. Each physiological sub-model may be developed and tested alone, or in combination with other physiological sub-models to simulate the whole physiological system, or parts thereof. In particular, one or more physiological sub-models may be tested for their impact on the overall mathematical model via sensitivity analysis or other mathematical suitable methods, and if the impact is limited one, or more sub-model, may be omitted in the overall physiological model, or only applied in some situations or circumstances, as it will be explained in more detail below.

In the context of the present invention, it's to be understood that the method may be used to - at least partly - compensate for the time delay from sampling to actual measurement of the blood sample by for example calculating backwards in time to obtain improved knowledge about the blood variable(s) at, or around, the actual time of sampling and thereby mitigate, reduce, indemnify, decrease, reduce, lessen and/or abate the effect of the said time delay (TD), and thereby correspondingly increase the knowledge, in particular the accuracy, about the blood variable(s) for use in any further medical and/or diagnostic procedure for a subject, such as a human patient in, or near, a clinical environment.

The blood variables relevant in context of the present invention may be conventionally measured by a blood gas measurement apparatus or system as the skilled person in blood measurements will readily understand, such as systems from Radiometer, Roche, Abbot Laboratories, GE Healthcare, Medtronic, Siemens Healthineers, Masino, Philips Healthcare, Nova Biomedical etc. However, to the best of the inventors' knowledge none of these manufacturers have the ability to compensate or mitigate for any significant time delay from sampling to measurement of the blood sample using a mathematical model with various physiological sub-models like the present invention, where time development of blood variables over for example 0.5-3 hours can be simulated with a high degree of reliability and accuracy.

In one advantageous embodiment, the first sub-model may describe red blood cell metabolism as a rate of strong acid production in whole blood ($\Delta$Acid), preferably with a rate of change of lactate concentration ($\Delta$LA) in whole blood being substantially equal to the acid production, cf. equation 1 below in the detailed description. Furthermore, the first sub-model describing red blood cell metabolism further may assume that glucose diffuses passively between plasma and RBCs so that concentrations can be assumed to be substantially equal in the plasma and the erythrocyte fractions of blood, cf. equation 3 below in the detailed description and that plasma glucose concentration changes in the opposite direction to lactate and are half that of the rate of lactate change.

In another advantageous embodiment, the second sub-model describing the effect of said red blood cell metabolism on the whole blood acid-base chemistry may comprise one, or more, of the following elements:

- the plasma and red blood cell buffering including

    - mass conservation equations,
    - mass action/reaction equations,
    - physiochemical properties,

- an empirical or biochemical relationship for the link between plasma and erythrocyte pH and/or,
- a description of plasma and erythrocyte fractions from haemoglobin concentration.

These elements will be further explained in connection with Figure 2 in the detailed description below.

In one beneficial embodiment, the second sub-model describing the effect of said red blood cell metabolism on the whole blood acid-base chemistry may comprise incorporating the effect of acid addition to whole blood by modifying the total buffer base concentration (BB), or strong ion difference (SID), and reducing this according to the acid addition ($\Delta$Acid) at a point in time so as to calculate one, or more, values of oxygenation and acid-base status according to, or substantially according, to the equation:

$$pH_p, PCO_{2,p}, PO_{2,p}, SO_{2,p} = acid\ base\ model(\ BB - \Delta\text{Acid}, tCO_2, tO_2, \text{tHb}, \text{Atot}),$$

wherein the acid base model refers to a biochemical mathematical model of the acid-base chemistry of blood as the skilled person will readily understand.

In another advantageous embodiment, the mathematical model may comprise one, or more, model parameter(s) chosen from the group consisting of:

- the total non-bicarbonate buffer, Atot,
- the red blood cell (RBC) acid production ($\Delta$Acid),
- a parameter describing the degree to which Cl$^-$ reduction occurs in the blood sample (CLmp), and
- a water distribution fraction,

preferably said one, or more, model parameter(s) being substantially unchanged from one subject to another. This is particularly advantageous in that these parameters are not so sensitive to specific patient details or differences, which may facilitate quicker modelling when implemented at a clinic, or a hospital, with a relatively large number of blood samples to be measured and where the time delay also is to be compensated. Nevertheless, it's contemplated that one, or more, of the said model parameter(s) may depend on other characteristics, and hence may vary over time. This could for example be due to differences in plasma protein concentrations, which may affect Atot, abnormalities in the red blood cell which may alter metabolic rate, or changes in osmolarity which may modify water distribution between the fractions of blood.

In yet another advantageous embodiment, the mathematical model may take into account a dependency on temperature of the sample during the time delay (TD), preferably a known or an estimated variable temperature profile during the time delay. Beneficially, the present invention may compensate for temperature including variations in temperature during the time delay. For example, the red blood cell (RBC) can have an acid production being dependent on blood temperature, and solubility coefficients for gasses in blood are known to be temperature dependent.

In another preferred embodiment, the mathematical model may further comprise a sub-model describing electrolyte distribution between plasma and erythrocyte fractions.

In yet another preferred embodiment, the computer-implemented method may further comprise a sub-model describing electrical neutrality in plasma, constraining concentrations of cations and anions to ensure equivalence and hence neutrality.

In other preferred embodiments, the mathematical model may further comprise a sub-model describing gas diffusion across plastic syringes with said blood sample. Where other materials of syringes than plastic are contemplated within the teaching and principle of the present invention, most syringes are currently made of plastic. Preferably, the sub-model describing gas diffusion across plastic syringes may comprise a relationship between the rate of acid production ($\Delta$Acid) and the transport of $CO_2$ over the syringe wall quantified as a rate of change in the total $CO_2$ concentration ($\Delta tCO_2$).

In other advantageous embodiments said time delay (TD) from sampling to actual measurement may be larger than 20 min., 30 min., 40 min., 50 min., 60 min., 70 min., 80 min., 90 min., 100 min., 110 min., 120 min, 150 min., or 180 min. Possibly it may be even longer i.e. more than 3 hours, for example up to 4, 5 or 6 hours. It is however contemplated that there may be an upper limit to the present invention in that mathematical model when there is too much acid produced, there is an expectation that the red blood cells (RBC) will not long have any metabolism, which thus may effectively set an upper time limit to how far the present invention may be used to describe or predict time development of some blood variables, and hence be used for compensate or mitigate a time delay (TD) according to the present invention.

In yet other advantageous embodiments the set of blood variables (BV) may be chosen from the group consisting of: pH, $PCO_2$, $PO_2$, $SO_2$, Hb, FMetHb, COHb, glucose, lactate, Na$^+$, Cl$^-$, Ca$^{2+}$, and/or K$^+$, and other blood variables readily available for the skilled person in blood gas measurements. It of course to be understood that the mentioning of chemical species, such as glucose, lactate, Na$^+$, Cl$^-$, or Ca$^{2+}$, etc. in the context of blood gas measurements are normally meant as concentration of these species, e.g. mmol/litre or other suitable chemical units such as charge equivalents e.g. meq/l, often indicated in chemistry by square brackets, i.e. [Na$^+$], or [Cl$^-$], as the skilled person in biochemistry, in particular blood gas measurements, will immediately understand.

In yet other valuable embodiments, the plurality of sub-models may be simplified, or their number reduced, in the sense that only a limited number of the physiological equations and elements are implemented as compared to the full extended model presented in the detailed description of an embodiment below. Thus, based on the insights and experience from testing and/or modelling of the present invention a number of situations may readily be identified for such reduced and simplified modelling, in particular if only a relatively small number of blood variable are of interest for modelling in time. Some examples include:

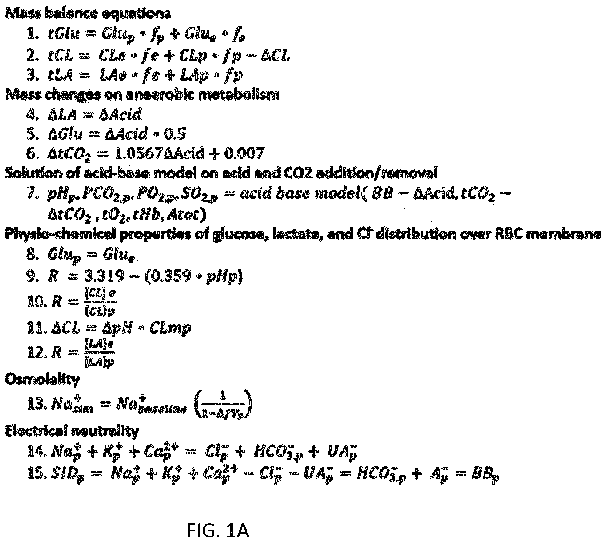

Example 1: If the effects of acid production on blood gas and acid-base are required then equation 7, figure 1A with suitable parameter values of $\Delta$Acid and Atot, along with a mathematical model of acid-base may be a sufficient model subset.

Example 2: If the effects on plasma glucose concentration alone is required then equations 1,5 and 8, figure 1A along with a suitable parameter value of $\Delta$Acid may be a sufficient model subset.

In other valuable embodiments, the mathematical model may calculate backwards in time from blood variables (BV'[Tm]) in the measured blood to blood variables (BV[Ts]) in the sampled blood. This is highly advantageous in that the time delay may be extended, e.g. up to several hours, and still it will be possible to apply the present invention to calculate backwards in the time to obtain an accurate value for one, or more, blood variable(s), where the time development is calculated backwards in time to the actual sampling time of the blood from a subject. It is expected that the present invention could thereby significantly impact the way blood samples are being transported and stored in many clinics and hospitals, where currently only a much shorter time is usually allowed between sampling and measurement of the blood typically a maximum of 209 minutes.

In yet other valuable embodiments, the mathematical model may calculate forwards in time from blood variables (BV[Ts]) in the sampled blood to blood variables (BV'[Tm]) in the measured blood. Thus, the present invention may also be applied for example for testing how a blood sample may change during transport and/or storage before measurement, which may be relevant for quality control and documentation in laboratories working for clinics or hospitals.

In a second aspect, the invention relates to a computer-implemented system for compensating the effects of time delay (TD) between sampling and actual measurement of acid-base, oxygenation and/or electrolyte status in a blood sample from an associated subject, the system being arranged for:

- receiving a blood sample with a known or estimated time of sampling (Ts),

- performing a blood gas measurement of said blood sample at a later time of measurement (Tm) resulting in a set of blood variables (BV'[Tm]) indicative of acid-base, oxygenation and/or electrolyte status in said blood sample,

- operating a mathematical model based on a plurality of physiological sub-models simulating time development in blood, the mathematical model comprising:

    o a first sub-model describing red blood cell metabolism, and
    o a second sub-model describing the effect of said red blood cell metabolism on the whole blood acid-base chemistry, and

- applying said mathematical model to calculate from the time of measurement (Tm) to the time of sampling (Ts) a modified set of blood variables (BV[Ts]) indicative of the corresponding acid-base, oxygenation and/or electrolyte status of said blood sample so as to at least partly compensate for said time delay (TD) between sampling and actual measurement.

Advantageously, the computer-implemented system according to the second aspect of the invention may be implemented in connection with a known blood gas measurement apparatus or system capable of measuring one, or more, blood variable(s), whereby the present invention may be enable or facilitate that the one, or more, blood variable may be calculated backwards in time to, or around, the time of sampling the blood sample from a subject, such as a human patient, and thereby increase the accuracy of blood gas measurements.

In a third aspect, the invention relates to a computer program product being adapted to enable a computer system comprising at least one computer having data storage means in connection therewith to control an system according to the second aspect of the invention, such as a computer program product comprising instructions which, when the program is executed by a computer, cause the computer to carry out the steps of the method of the invention according to the first aspect, the invention thereby being computer-implemented.

This aspect of the invention is particularly, but not exclusively, advantageous in that the present invention may be accomplished by a computer program product enabling a computer system to carry out the operations of the apparatus/system of the first aspect of the invention when down- or uploaded into the computer system. Such a computer program product may be provided on any kind of computer readable medium, or through a network.

In a fourth aspect, the invention relates to the use of the method and/or the system according to the first and/or the second aspect for compensating the effects of time delay (TD) between sampling and actual measurement of acid-base, oxygenation and/or electrolyte status in a blood sample from a subject.

In a preferred embodiment, preferably based on a human sample or veterinary sample, the subject is a mammal, preferably a human, preferably use wherein the subject is a healthy subject or a subject with a disease, such as respiratory diseases, cardiovascular diseases or metabolic diseases, causing low oxygen levels, abnormal carbon dioxide levels, and/or electrolyte abnormalities.

The use of a method according to the first aspect or a system according to the second aspect, for estimating a blood variable in a blood sample at the timepoint the sample has been sampled from a subject, wherein the timepoint for estimating the level is at a later timepoint than the timepoint of sampling.

The use wherein the location of obtaining the sample is at a different location than the location of estimating the blood variable, such as in different buildings or rooms.

The use according to a method of the first aspect, wherein the time between obtaining the blood sample and the time of estimating is in the range 1 minute to 3 hours, such as 30 min to 2 hours, such as 1 hour.

Use of a method according to the first aspect or a system according to the second aspect, wherein the subject is a mammal, preferably a human. In an embodiment, the mammal is selected from the group consisting of a pet, such as a dog or cat, a racing animal such as a horse, dog or camel, or a farm animal, such as a cow, goat, camel, or horse.

The use wherein the subject is a healthy subject or a subject with a disease, such as respiratory diseases, cardiovascular diseases or metabolic diseases, causing low oxygen levels, abnormal carbon dioxide levels, and/or electrolyte abnormalities.

In an embodiment, the disease is selected from the group consisting of:

Chronic Obstructive Pulmonary Disease (COPD), asthma, pneumonia, pulmonary embolism, pulmonary fibrosis, congestive heart failure, sleep apnea, anemia,
Acute Respiratory Distress Syndrome (ARDS), and COVID-19, but not limited to any these specific disease.

The individual aspects of the present invention may each be combined with any of the other aspects. These and other aspects of the invention will be apparent from the following description with reference to the described embodiments.

BRIEF DESCRIPTION OF THE FIGURES

The method according to the invention will now be described in more detail with regard to the accompanying figures. The figures show one way of implementing the present invention and is not to be construed as being limiting to other possible embodiments falling within the scope of the attached claim set.

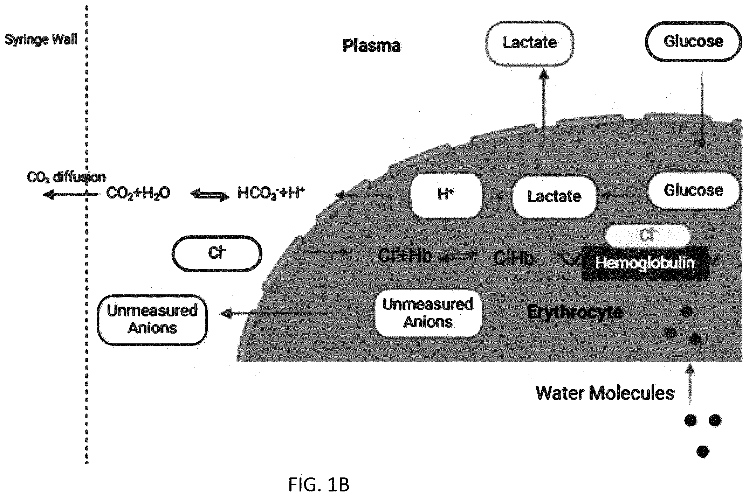

Figure 1A and 1B show equations and physiochemical processes describing the delay in analysis of the blood including (equation numbers from figure), mass balance/conservation (1-3), mass changes during anaerobic metabolism of red blood cells (4-5) and resulting changes in carbon dioxide due to diffusion over the syringe wall (6), integration of the mathematical model of acid-base chemistry into the method presented here (7), physiochemical properties due to the RBC membrane (8-12), the effects of water transport on Na+ concentration (13) and electrical neutrality (14 and 15),

Figure 2A and 2B show the mathematical model of acid-base chemistry of the blood, the model includes chemical reactions describing bicarbonate and non-bicarbonate buffering in plasma and erythrocyte fractions, including the competitive binding of $H^+$, $O_2$ and $CO_2$ to haemoglobin. Equations describe the mass balance/conservation of state variables (1-5r), mass action equations describing chemical reactions (6r-17r) and physiochemical properties including gas solubility (18r to 21r), blood fractions (22r, 23r) and pH differences across the RBC membrane (24r), and the model includes calculation of the base excess (BE) (25r), and equations representing two different methods to account for electrical neutrality in the blood (26ar, 26br),

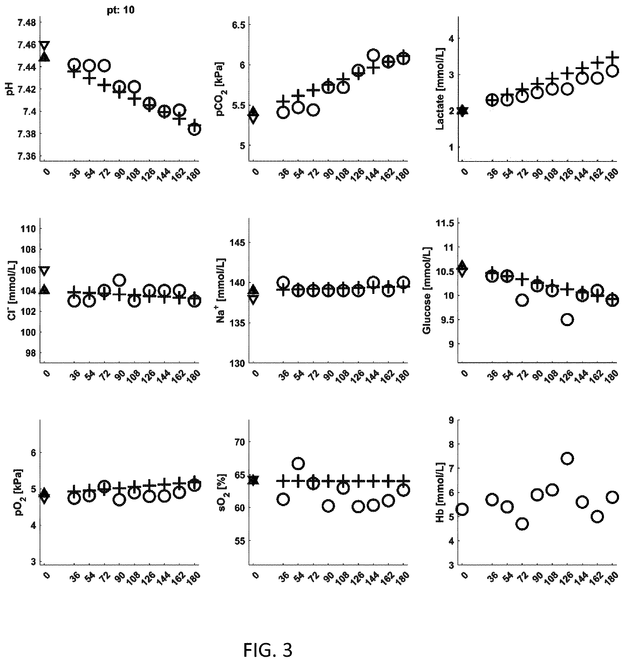

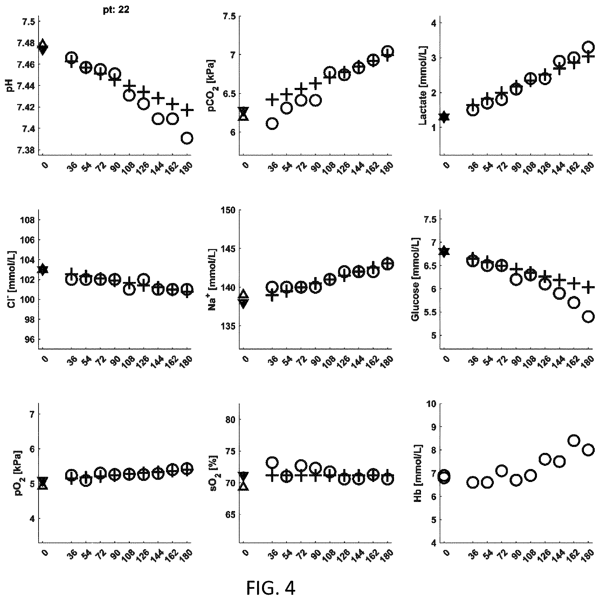

Figures 3 and 4 show plots illustrating baselines measurements (triangles), raw data (circles) and model fits (crosses) for two representative patients, patient 10 and patient 22 of data describing 30 patient samples, respectively, over the time period of 0 to 180 minutes, and

Figure 5 is a schematic system-chart representing an out-line of/in detail the operations of the method and/or the computer program product according to the invention.

DETAILED DESCRIPTION OF AN EMBODIMENT

The following text describes a detailed embodiment of the invention including description of the mathematical equations of figure 1A. In addition, it describes the application of this embodiment in two patient cases, the data of which is illustrated in figures 3 and 4. These data are drawn from a clinical study in 30 critically ill patients and exemplify use of the method. For each of these patients 10 blood samples of 1mL were collected in series within 4 minutes. Each of these blood samples were analysed to measure values of pH, pCO2, pO2, SO2, Hb, FMetHb, COHb, glucose, lactate, $Na^+$, $Cl^-$, $Ca^{2+}$, $K^+$. The samples were analysed at different time points, 0, 36, 54, 72, 90, 108, 126, 144, 162, and 180 minutes, respectively to allow for calculation of values forwards and backwards in time. All samples were drawn into syringes which are routinely used in the clinical analysis of acid-base, oxygenation and electrolyte status and kept at room temperature.

These mathematical equations which can be used to calculate from one timepoint to another in a forwards or backwards direction are illustrated in figure 1A and 2B. These equations describe the necessary biochemical processed to perform these calculation/simulations, as follows.

Red blood cells (RBCs) undergo anaerobic metabolism producing strong acid and lactate in equivalent amounts. The rate of strong acid production in whole blood (ΔAcid) is applied as a parameter allowing the calculation of the rate of change of lactate concentration in whole blood ($\Delta LA$) from equation 4 of figure 1A.

$$\Delta LA = \Delta \text{Acid} \qquad (4)$$

When glucose is used in anaerobic metabolism each mmol of glucose used produces 2 mmol strong acid and lactate. Glucose utilization ($\Delta Glu$) is therefore described using equation 5 of figure 1A

$$\Delta Glu = -\Delta Acid * 0.5 \qquad (5)$$

Passive diffusion of glucose between plasma means that concentrations can be assumed to be equivalent (figure 1A equation 8). Total glucose concentration in the whole blood can be described as the sum of the concentration is plasma and erythrocyte weighted by their respective fractions (figure 1A, equation 1)

$$Glu_p = Glu_e \qquad (8)$$

$$tGlu = Glu_p * f_p + Glu_e * f_e \quad (1)$$

The production of acid by the red blood cells during metabolism ($\Delta$Acid) will mean that conditions in the blood will change. To simulate such conditions requires a mathematical model of the acid-base chemistry of plasma and erythrocyte fraction of blood. Here we apply the mathematical model of Rees and Andreassen, cf. Rees SE, Andreassen S. Mathematical Models of Oxygen and Carbon Dioxide Storage and Transport: The Acid-Base Chemistry of Blood. Crit Rev Biomed Eng. 2005;33(3):209-64.

This model, illustrated in Figure 2A and 2B, describes the plasma and red blood cell buffering including mass conservation equations, mass action/reaction equations, physiochemical properties, an empirical relationship for the link between plasma and erythrocyte pH representing the Donnan effect, and description of plasma and erythrocyte fractions from hemoglobin concentration.

This model can be applied to simulate the effects of acid addition to whole blood due to red blood cell metabolism. This is performed by simulating a modification of the total buffer base concentration (BB) of the blood. If acid is added, as occurs in simulations forward in time this will reduce BB. If acid is removed, as occurs in simulations backward in time this will increase BB. Solution of the whole model of Rees and Andreassen in figures 2A and 2B on addition or removal of acid is summarized in equation 7 of figure 1A

$$pH_p, PCO_{2,p}, PO_{2,p}, SO_{2,p} = acid\ base\ model(\ BB - \Delta\text{Acid}, tCO_2, tO_2, \text{tHb}, \text{Atot}) \quad (7)$$

Plastic syringes are permeable to $O_2$ and $CO_2$, and delay in analysis may result in gas transport over the syringe wall. $O_2$ diffusion is possible but not included in this specific embodiment. In the data from the 30 patients no $O_2$ diffusion was necessary for accurate simulations.

For $CO_2$ diffusion, partial pressures of $CO_2$ are higher than atmospheric air and diffusion is likely due to a $CO_2$ gradient across the syringe wall. In addition, $CO_2$ partial pressures will remain high during the delay period, with acid production causing $CO_2$ dissociation from bicarbonate. In this embodiment we described this diffusion as a loss of $CO_2$ ($\Delta tCO_2$) which was related to the acid production ($\Delta$Acid) using a linear fit to the data of our 30 patients. This linear fit resulted in equation 6 of figure 1A, below, although other empirical or biochemical models describing this diffusion dependent on the conditions or syringe may be possible,

$$\Delta tCO_2 = -1.0567\Delta Acid + 0.007 \qquad (6)$$

If diffusion exists a reformulated summary equation of the model of Rees and Andreassen (Figure 1A, equation 7) can be used to solve this model accounting for the change in carbon dioxide ($\Delta tCO_2$)

$$pH_p, PCO_{2,p}, PO_{2,p}, SO_{2,p} = acid\ base\ model(\ BB - \Delta\text{Acid}, tCO_2 + \Delta tCO_2, tO_2, \text{tHb}, \text{Atot}) \quad (7)$$

Changes in acid-base status in blood will modify electrolyte distribution between plasma and red blood cells. A number of equations are used to account for this.

Chloride (Cl⁻) is modelled as the Donnan effect across the red blood cell membrane with the ratio (R) of Cl⁻ in erythrocyte (e) and plasma fractions represented in equation 10 (figure 1A). This ratio is known to be pH dependent, which is modelled using equation 11 (figure 1A), with the total mass of charged Cl⁻ accounted for by the mass balance equation 2 (figure 1A). As Cl⁻ can bind to haemoglobin in red blood cells which removes its charge, an equation is used to account for a reduction in the total of charged Cl⁻ ($\Delta CL$) (equation 11, figure 1 A) with this assumed here to be dependent on the pH change (($\Delta$pH) from the initial condition, i.e. the time point at the start of simulation. The equation described here and illustrated in figure 1A are

$$R = \frac{[CL]\,e}{[CL]\,p} \qquad (10)$$

$$R = 3.319 - (0.359 * pHp) \qquad (9)$$

$$tCL = CLe * fe + CLp * fp - \Delta CL \qquad (2)$$

$$\Delta CL = \Delta pH * CLmp \qquad (11)$$

Lactate distribution across the red blood cell membrane is described in an equation similar to Cl⁻, (equation 12, figure 1A), using the same ratio describing the Donnan effect. Total lactate is represented as the weighted sum of concentrations in plasma and red blood cells (equation 3, figure 1A).

$$R = \frac{[LA]\,e}{[LA]\,p} \qquad (12)$$

$$tLA = LAe * fe + LAp * fp \qquad (3)$$

Other electrolytes usually measured in plasma include Na⁺, K⁺, Ca²⁺. Values of K⁺, and Ca²⁺ are relatively small and any changes are unlikely to be distinguished from measurement noise such that equations for these are not considered here. Values of Na⁺ are tightly regulated, with almost all Na⁺ remaining in the plasma compartment. Changes are therefore primarily due to water transport between plasma and erythrocyte fractions due to osmolarity. Equation 13 (figure 1A) accounts for this simulating Na⁺ concentration ( $Na^+_{sim}$ ) is calculated from baseline values ( $Na^+_{baseline}$ ) according to a change in the fraction of plasma volume ($\Delta fV_P$) per hour (equation 13).

$$Na^+_{sim} = Na^+_{baseline} \left( \frac{1}{1 - \Delta fV_P} \right) \qquad (13)$$

An additional equation can be used to describe electrical neutrality in the plasma (equation 14, figure 1 A), such that the sum of cations and anions is equivalent. By equating the difference in measured strong cations and anions - the strong ion difference (SID), with the blood buffer base (BB) calculations can be performed of any unmeasured anions ( $UA^-_p$ ) (equation 15 figure 1A). This calculation is not necessary to perform simulations backwards and forwards in time, but can be used to understand any unknown transport of strong cations between red blood cells and plasma

$$Na^+_p + K^+_p + Ca^{2+}_p = Cl^-_p + HCO^-_{3,p} + UA^-_p \qquad (14)$$

$$SID_p = Na^+_p + K^+_p + Ca^{2+}_p - Cl^-_p - UA^-_p = HCO^-_{3,p} + A^-_p = BB_p \qquad (15)$$

Equation 15 also includes $A^-_p$ which is the non-bicarbonate buffer concentration.
This can be estimated if albumin or total plasma protein concentration are known allowing estimation of the value of the sum of the weak acid and weak base forms of the non-bicarbonate buffers (*Atot*). *Atot* can also be estimated from equations 14 and 15 assuming $UA^-_p$ to be zero, and it is this approach that is used in the two patient cases simulated in figures 3 and 4.
The 15 equations, an initial blood gas measurement and values of the 4 model parameters can then simulate forwards or backwards in time to any time point. Model parameters are the total concentration of non-bicarbonate conjugate acid and base (*Atot*), the anaerobic acid production ($\Delta$Acid), chloride binding to hemoglobin *(CLmp)* and the change in the fraction of plasma volume ($\Delta fV_P$). In the 30 patients described here values of model parameters were: *Atot* - 7.8(9.7) meq/l (median,IQR), $\Delta$Acid - constant at 0.008 meq/l/min, *CLmp*- 29(35) mmol/l (median, IQR), $\Delta fV_P$ - 0.0082(0.0076) fraction/hour.

Figures 3 and 4 illustrate two example patients representative of the 30 currently studies. Circles are measured data and crosses are model simulations performed forwards in time from time point zero, using measurements taken at this time, values of model parameters and model equations. For these patients, the mathematical model can thus describe the time development of these blood variables.

Figure 5 is a schematic system-chart representing an out-line of/in detail the operations of the method and/or the computer program product according to the invention. Thus, the invention relates to a computer-implemented method for compensating the effects of time delay (TD) between sampling and actual measurement of acid-base, oxygenation and/or electrolyte status in a blood sample from a subject, the method comprising:

**S1** providing a blood sample with a known or estimated time of sampling (Ts), such as a blood sample obtained from a patient in a clinic or a hospital,

**S2** performing a blood gas measurement of said blood sample at a later time of measurement (Tm) resulting in a set of blood variables (BV'[Tm]) indicative of acid-base, oxygenation and/or electrolyte status in said blood sample,

**S3** providing a mathematical model, such as the model shown in Figures 1-2, based on a plurality of physiological sub-models simulating time development in blood, the mathematical model comprising:

o a first sub-model describing red blood cell metabolism, and
o a second sub-model describing the effect of said red blood cell metabolism on the whole blood acid-base chemistry, and

**S4** applying said mathematical model to calculate from the time of measurement (Tm) to the time of sampling (Ts) a modified set of blood variables (BV[Ts]) indicative of the corresponding acid-base, oxygenation and/or electrolyte status of said blood sample so as to at least partly compensate for said time delay (TD) between sampling and actual measurement, cf. Figures 3 and 4 for time simulations of some blood variables for two patients.

In short, the invention relates to a method for compensating the effects of time delay (TD) between sampling and actual measurement of acid-base, oxygenation and/or electrolyte status in a blood sample from a subject, such as a human patient. A mathematical model is simulating time development in the blood sample, the mathematical model having 1) a first sub-model describing red blood cell metabolism, and 2) a second sub-model describing the effect of the red blood cell metabolism on the whole blood acid-base chemistry. The mathematical model calculates from a time of measurement (Tm) to the time of sampling (Ts) a modified set of blood variables to compensate for a time delay (TD), e.g. by calculating backwards in time 0.5-3 hours. The invention can simulate the biochemical processes occurring in blood, and demonstrates the ability to simulate changes in measured blood variables over time. This may simplify transport and/or storage constraints of blood samples, and improve quality and accuracy of blood gas measurements, cf. Figure 3.

The invention can be implemented by means of hardware, software, firmware or any combination of these. The invention or some of the features thereof can also be implemented as software running on one or more data processors and/or digital signal processors.

The individual elements of an embodiment of the invention may be physically, functionally and logically implemented in any suitable way such as in a single unit, in a plurality of units or as part of separate functional units. The invention may be implemented in a single unit, or be both physically and functionally distributed between different units and processors.

Although the present invention has been described in connection with the specified embodiments, it should not be construed as being in any way limited to the presented examples. The scope of the present invention is to be interpreted in the light of the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

**Claims**

1. A computer-implemented method for compensating the effects of time delay (TD) between sampling and actual measurement of acid-base, oxygenation and/or electrolyte status in a blood sample from a subject, the method comprising:

   - providing a blood sample with a known or estimated time of sampling (Ts),
   - performing a blood gas measurement of said blood sample at a later time of measurement (Tm) resulting in a set of blood variables (BV'[Tm]) indicative of acid-base, oxygenation and/or electrolyte status in said blood sample,
   - providing a mathematical model based on a plurality of physiological sub-models simulating time development in blood, the mathematical model comprising:

      o a first sub-model describing red blood cell metabolism, and
      o a second sub-model describing the effect of said red blood cell metabolism on the whole blood acid-base chemistry, and

   - applying said mathematical model to calculate from the time of measurement (Tm) to the time of sampling (Ts) a modified set of blood variables (BV[Ts]) indicative of the corresponding acid-base, oxygenation and/or electrolyte status of said blood sample so as to at least partly compensate for said time delay (TD) between sampling and actual measurement.

2. The computer-implemented method according to any of the preceding claims, wherein the first sub-model describes red blood cell metabolism as a rate of strong acid production in whole blood ($\Delta$Acid), preferably with a rate of change of lactate concentration ($\Delta$LA) in whole blood being substantially equal to the acid production.

3. The computer-implemented method according to claim 2, wherein the first sub-model describing red blood cell metabolism further assumes that glucose diffuses passively between plasma and RBCs so that concentrations can be assumed to be substantially equal in the plasma and the erythrocyte fractions of blood.

4. The computer-implemented method according to any of the preceding claims, wherein the second sub-model describing the effect of said red blood cell metabolism on the whole blood acid-base chemistry comprises one, or more, of the following elements:

   - the plasma and red blood cell buffering including

      - mass conservation equations,
      - mass action/reaction equations, and
      - physiochemical properties,

   - an empirical or biochemical relationship for the link between plasma and erythrocyte pH and/or,
   - a description of plasma and erythrocyte fractions from haemoglobin concentration.

5. The computer-implemented method according to any of the preceding claims, wherein the second sub-model describing the effect of said red blood cell metabolism on the whole blood acid-base chemistry comprises incorporating the effect of acid addition to whole blood by modifying the total buffer base concentration (BB), or strong ion difference (SID), and reducing this according to the acid addition ($\Delta$Acid) at a point in time so as to calculate one, or more, values of oxygenation and acid-base status according to, or substantially according, to the equation:

$$pH_p, PCO_{2,p}, PO_{2,p}, SO_{2,p} = acid\ base\ model(\ BB - \Delta\text{Acid}, tCO_2, tO_2, \text{tHb}, \text{Atot}),$$

wherein the acid base model refers to a biochemical mathematical model of the acid-base chemistry of blood.

6. The computer-implemented method according to any of the preceding claims, wherein the mathematical model comprises one, or more, model parameter(s) chosen from the group consisting of:

   - the total non-bicarbonate buffer, Atot,
   - the red blood cell (RBC) acid production ($\Delta$Acid),

- a parameter describing the degree to which Cl⁻ reduction occurs in the blood sample (CLmp), and
- a water distribution fraction.,

preferably said one, or more, model parameter(s) being substantially unchanged from one subject to another.

7. The computer-implemented method according to any of the preceding claims, wherein the mathematical model takes into account a dependency on temperature of the sample during the time delay (TD), preferably a known or an estimated variable temperature profile during the time delay.

8. The computer-implemented method according to any of the preceding claims, wherein the mathematical model further comprises a sub-model describing electrolyte distribution between plasma and erythrocyte fractions.

9. The computer-implemented method according to any of the preceding claims, wherein the mathematical model further comprises a sub-model describing gas diffusion across plastic syringes with blood sample.

10. The computer-implemented method according to any of the preceding claims, wherein said time delay (TD) from sampling to actual measurement is larger than 20 min., 30 min., 40 min., 50 min., 60 min., 70 min., 80 min., 90 min., 100 min., 110 min., 120 min, 150 min., or 180 min.

11. The computer-implemented method according to any of the preceding claims, wherein the set of blood variables (BV) are chosen from the group consisting of: pH, $PCO_2$, $PO_2$, $SO_2$, Hb, FMetHb, COHb, glucose, lactate, Na⁺, Cl⁻, Ca²⁺, and/or K⁺.

12. The computer-implemented method according to any of the preceding claims, wherein the mathematical model calculates backwards in time blood variables (BV'[Tm]) in the measured blood to blood variables (BV[Ts]) in the sampled blood.

13. The computer-implemented method according to any of the preceding claims, wherein the mathematical model calculates forwards in time from blood variables (BV[Ts]) in the sampled blood to blood variables (BV'[Tm]) in the measured blood.

14. A computer-implemented system for compensating the effects of time delay (TD) between sampling and actual measurement of acid-base, oxygenation and/or electrolyte status in a blood sample from an associated subject, the system being arranged for:

- receiving a blood sample with a known or estimated time of sampling (Ts),
- performing a blood gas measurement of said blood sample at a later time of measurement (Tm) resulting in a set of blood variables (BV'[Tm]) indicative of acid-base, oxygenation and/or electrolyte status in said blood sample,
- operating a mathematical model based on a plurality of physiological sub-models simulating time development in blood, the mathematical model comprising:

o a first sub-model describing red blood cell metabolism, and
o a second sub-model describing the effect of said red blood cell metabolism on the whole blood acid-base chemistry, and

- applying said mathematical model to calculate from the time of measurement (Tm) to the time of sampling (Ts) a modified set of blood variables (BV[Ts]) indicative of the corresponding acid-base, oxygenation and/or electrolyte status of said blood sample so as to at least partly compensate for said time delay (TD) between sampling and actual measurement.

15. A computer program product being adapted to enable a computer system comprising at least one computer having data storage means in connection therewith to control a system according to claim 14, such as a computer program product comprising instructions which, when the program is executed by a computer, cause the computer to carry out the steps of the method of claim 1.

16. The use of the method and/or the system according to any of claims 1-15 for compensating the effects of time delay (TD) between sampling and actual measurement of acid-base, oxygenation and/or electrolyte status in a blood sample from a subject.

**Mass balance equations**

1. $tGlu = Glu_p \cdot f_p + Glu_e \cdot f_e$
2. $tCL = CLe \cdot fe + CLp \cdot fp - \Delta CL$
3. $tLA = LAe \cdot fe + LAp \cdot fp$

**Mass changes on anaerobic metabolism**

4. $\Delta LA = \Delta Acid$
5. $\Delta Glu = \Delta Acid \cdot 0.5$
6. $\Delta tCO_2 = 1.0567\Delta Acid + 0.007$

**Solution of acid-base model on acid and CO2 addition/removal**

7. $pH_p, PCO_{2,p}, PO_{2,p}, SO_{2,p} = acid\ base\ model(\ BB - \Delta Acid, tCO_2 - \Delta tCO_2, tO_2, tHb, Atot)$

**Physio-chemical properties of glucose, lactate, and Cl⁻ distribution over RBC membrane**

8. $Glu_p = Glu_e$
9. $R = 3.319 - (0.359 \cdot pHp)$
10. $R = \dfrac{[CL]e}{[CL]p}$
11. $\Delta CL = \Delta pH \cdot CLmp$
12. $R = \dfrac{[LA]e}{[LA]p}$

**Osmolality**

13. $Na^+_{sim} = Na^+_{baseline}\left(\dfrac{1}{1-\Delta fV_p}\right)$

**Electrical neutrality**

14. $Na^+_p + K^+_p + Ca^{2+}_p = Cl^-_p + HCO^-_{3,p} + UA^-_p$
15. $SID_p = Na^+_p + K^+_p + Ca^{2+}_p - Cl^-_p - UA^-_p = HCO^-_{3,p} + A^-_p = BB_p$

FIG. 1A

FIG. 1B

FIG. 2A

**Mass balance equations**

1r) $tCO_2 = (CO_{2,p} + HCO_{3,p}^-) f_p + (CO_{2,e} + HCO_{3,e}^- + HbNHCOO^- + HbO_2NHCOO^-) f_e$

2r) $Atot_p = A_p^- + HA_p$

3ar) $Hb = (HbNH_3^+ + HbNH_2 + HbNHCOO^- + HbO_2NH_3^+ + HbO_2NH_2 + HbO_2NHCOO) f_e$

3br) $Hb = (Hb(RH)_b + Hb(R^-)_b + HbO_2(RH)_b + HbO_2(R^-)_b) f_e$

4ar) $SID = BB_p = HCO_{3,p}^- + A_p^-$

4br) $SID_e = BB_e = HCO_{3,e}^- + b Hb(R^-)_b + b HbO_2(R^-)_b + HbNH_2 + HbO_2NH_2 + 2 HbNHCOO^- + 2 HbO_2NHCOO^-.$

4cr) $BB = BB_p f_p + BB_e f_e$

5r) $tO_2 = O_{2,p} f_p + (O_{2,e} + HbO_2NH_3^+ + HbO_2NH_2 + HbO_2NHCOO^-) f_e$

**Mass action equations**

6r) $pH_p = pKHCO_{3,p} + log_{10}(HCO_{3,p}^- / CO_{2,p})$

7r) $pH_p = pKa_p + log_{10}(A_p^- / HA_p)$

8r) $pH_e = pKHCO_{3,e} + log_{10}(HCO_{3,e}^- / CO_{2,e})$

9r) $pH_e = pKzd + log_{10}(HbNH_2 / HbNH_3^+)$

10r) $pH_e = pKzo + log_{10}(HbO_2NH_2 / HbO_2NH_3)$

11r) $pH_e = pKcd + log_{10}(HbNHCOO^- / (HbNH_2 \; CO_{2,e}))$

12r) $pH_e = pKco + log_{10}(HbO_2NHCOO^- / (HbO_2NH_2 \; CO_{2,e}))$

13r) $pH_e = pKzd_R + log_{10}(Hb(R^-)_b / Hb(RH)_b)$

14r) $pH_e = pKzo_R + log_{10}(HbO_2(R^-)_b / HbO_2(RH)_b)$

15r) $SO_2 = ODC( PO_2, pH, PCO_2,DPG)$

16r) $SO_2 = (HbO_2NH_3^+ + HbO_2NH_2 + HbO_2NHCOO^-) / Hb_e$

17r) $SO_2 = (HbO_2(RH)_b + HbO_2(R^-)_b) / Hb_e$

**Physico-chemical properties**

18r) $O_{2,p} = \alpha_{O2} \; PO_2$

19r) $O_{2,e} = \alpha_{O2} \; PO_2$

20r) $CO_{2,p} = \alpha_p \; PCO_2$

21r) $CO_{2,e} = \alpha_e \; PCO_2$

22r) $f_p = 1 - f_e$

23r) $f_e = Hb/21$

24r) $pH_e = 7.19 + 0.77 \; (pH_p - 7.4) + 0.031 \; \delta sO_2$

**Siggaard-Andersen (BE and anion gap)**

25r) $BE = BB - nBB$

26ar) $A_p^- - X^- = Na_p^+ + K_p^+ + 2Ca_p^{++} + 2Mg_p^{++} - Cl_p^- - HCO_{3,p}^-$

**Stewart (SID)**

26br) $SID = BB_p = Na_p^+ + K_p^+ + 2Ca_p^{++} + 2Mg_p^{++} - Cl_p^- - X^-$

FIG. 2B

FIG. 3

FIG. 4

```
┌─────────────────────────────────────┐
│                 S1                   │
└─────────────────────────────────────┘
                   │
                   ▼
┌─────────────────────────────────────┐
│                 S2                   │
└─────────────────────────────────────┘
                   │
                   ▼
┌─────────────────────────────────────┐
│                 S3                   │
└─────────────────────────────────────┘
                   │
                   ▼
┌─────────────────────────────────────┐
│                 S4                   │
└─────────────────────────────────────┘
```

Fig. 5

Europäisches
Patentamt

European
Patent Office

Office européen
des brevets

## EUROPEAN SEARCH REPORT

**Application Number**

EP 24 21 2124

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MYBURGH A ET AL: "A physio-chemical mathematical model of the effects of blood analysis delay on acid-base, metabolite and electrolyte status: evaluation in blood from critical care patients", SOUTH AFR J ANAESTH ANALG, vol. 25, no. 3, 1 January 2019 (2019-01-01), pages 21-25, XP093267849, * abstract, title, introduction, p. 22-23, fig. 1, table I,II * | 1-16 | INV.<br>G16C60/00<br>G16B5/30<br>G01N33/49 |
| A | US 2016/357926 A1 (REES STEPHEN EDWARD [DK] ET AL) 8 December 2016 (2016-12-08) * the whole document * | 1-16 | |
| A | REES ET AL: "Mathematical Models of Oxygen and Carbon Dioxide Storage and Transport: The Acid-Base Chemistry of Blood", CRITICAL REVIEWA IN BIOMEDICAL ENGINEERING, vol. 33, no. 3, 1 January 2005 (2005-01-01), pages 209-264, XP093267760, * the whole document * | 1-16 | |
| X,P | NEVIRIAN: "A physio-chemical mathematical model of the effects of blood analysis delay on acid-base, metabolite and electrolyte status: evaluation in blood from critical care patients", CLIN CHME LAB MED, vol. aop, 31 December 2024 (2024-12-31), XP093267797, DOI: 10.1515/cclm-2024-1350 * the whole document * | 1 | |

**TECHNICAL FIELDS SEARCHED (IPC)**

G16C
G16B
G01N

The present search report has been drawn up for all claims

| Place of search | Date of completion of the search | Examiner |
|---|---|---|
| Munich | 9 April 2025 | Lüdemann, Susanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P4C01)

**EP 4 742 252 A1**

## ANNEX TO THE EUROPEAN SEARCH REPORT
## ON EUROPEAN PATENT APPLICATION NO.

EP 24 21 2124

This annex lists the patent family members relating to the patent documents cited in the above-mentioned European search report.
The members are as contained in the European Patent Office EDP file on
The European Patent Office is in no way liable for these particulars which are merely given for the purpose of information.

09-04-2025

| Patent document cited in search report | Publication date | Patent family member(s) | Publication date |
|---|---|---|---|
| US 2016357926 A1 | 08-12-2016 | CN 105960198 A | 21-09-2016 |
| | | DK 3076862 T3 | 23-09-2019 |
| | | EP 3076862 A1 | 12-10-2016 |
| | | ES 2742446 T3 | 14-02-2020 |
| | | US 2016357926 A1 | 08-12-2016 |
| | | US 2020411178 A1 | 31-12-2020 |
| | | US 2024339210 A1 | 10-10-2024 |
| | | WO 2015081965 A1 | 11-06-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

• US 6859738 B

### Non-patent literature cited in the description

• **REES SE** ; **ANDREASSEN S.** Mathematical Models of Oxygen and Carbon Dioxide Storage and Transport: The Acid-Base Chemistry of Blood.. *Crit Rev Biomed Eng.*, 2005, vol. 33 (3), 209-64